(19) Europäisches Patentamt
European Patent Office
Office européen des brevets

(11) Publication number: 0 127 356 B1

(12) EUROPEAN PATENT SPECIFICATION

(45) Date of publication of patent specification: 30.01.91

(21) Application number: 84303148.5

(22) Date of filing: 09.05.84

(51) Int. Cl.$^5$: C 07 D 211/58, C 07 D 401/14, C 08 K 5/34 // C08L23/12, C08L75/04, C08L27/06

# (54) A 2,2,6,6-tetramethylpiperidine derivative, its production and its use as a stabilizer for synthetic resins.

(30) Priority: 27.05.83 JP 94467/83
01.08.83 JP 141737/83

(43) Date of publication of application:
05.12.84 Bulletin 84/49

(45) Publication of the grant of the patent:
30.01.91 Bulletin 91/05

(84) Designated Contracting States:
CH DE FR GB IT LI NL

(56) References cited:
EP-A-0 031 304
EP-A-0 034 829
EP-A-0 047 605
EP-A-0 081 688
FR-A-2 366 278

The file contains technical information submitted after the application was filed and not included in this specification

(73) Proprietor: SUMITOMO CHEMICAL COMPANY, LIMITED
Kitahama 4-chome 5-33
Chuo-ku Osaka 541 (JP)

(72) Inventor: Yoshimura, Masakatsu
3-3-15 Ohama Kitamachi
Sakai Osaka-fu (JP)
Inventor: Fujii, Takeo
2-10-3, Sone Higashimachi
Toyonaka Osaka-fu (JP)
Inventor: Yachigo, Shinichi
2-11-7-305, Sone Higashimachi
Toyonaka Osaka-fu (JP)
Inventor: Ishii, Tamaki
1-12-2, Uchihonmachi Suita
Osaka-fu (JP)

(74) Representative: Geering, Keith Edwin et al
REDDIE & GROSE 16 Theobalds Road
London WC1X 8PL (GB)

EP 0 127 356 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to stabilisation of synthetic resins, and provides 2,2,6,6-tetramethylpiperidine derivatives represented by the formula (I),

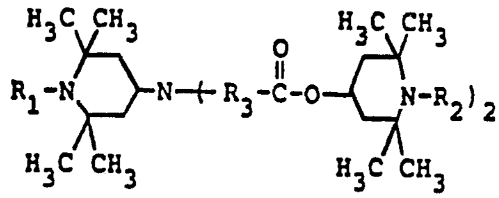

(I)

wherein $R_1$ and $R_2$ independently represent a hydrogen atom or methyl group, and $R_3$ represents an alkylene group having one to four carbon atoms, their production, and a stabilizer for synthetic resins containing it as an effective ingredient.

It is well known that synthetic resins such as polyethylene, polypropylene, polyvinyl chloride, polyurethane, and ABS resin deteriorate under the action of light, which can variously cause phenomena such as softening, embrittlement, and discoloration.

For preventing such deterioration due to light, the use of various photostabilizers is well known. Such photostabilizers include for example 2-hydroxy-4-methoxybenzophenone, 2-hydroxy-4-n-octoxybenzophenone, 2-(2-hydroxy-5-methylphenyl)benzotriazole, 2-(2-hydroxy-3-tert-butyl-5-methylphenyl)-5-chlorobenzotriazole, 2-(2-hydroxy-3,5-dipentylphenyl)benzotriazole, ethyl 2-cyano-3,3-diphenylacrylate, 2,4-di-tert-butylphenyl 3,5-di-tert-butyl-4-hydroxybenzoate, [2,2′-thiobis(4-tert-octylphenolate)]-n-butylamine·nickel(II), Ni salt of bis(3,5-di-tert-butyl-4-hydroxybenzylphosphoric acid)monoethyl ester, and bis(2,2,6,6-tetramethyl-4-piperidyl)sebacate. These photostabilizers, however, are not wholly satisfactory in terms of light fastness.

EP.0031304 discloses piperidinol carbamates of formula

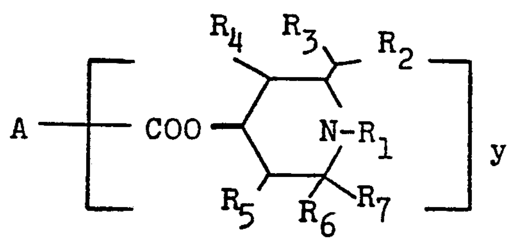

in which y is an integer from 1—4, and their use as light, heat and oxidation stabilisers for polymers.

EP 0034829 discloses tetra-alkyl-4-aminopiperidine-containing acids and their salts, together with their use as polymer stabilisers.

We have found that 2,2,6,6-tetramethylpiperidine derivatives of formula (I) are effective in resisting the degradation of synthetic resins by light. These derivatives according to the invention can be produced by reacting a 4-amino-2,2,6,6-tetramethylpiperidine compound represented by the formula (II),

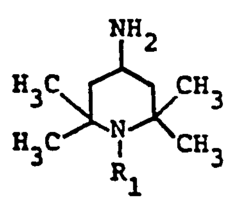

(II)

wherein $R_1$ represents a hydrogen atom or methyl group, with a halogenated carboxylic compound of the formula (III),

$$X—R_3—\overset{O}{\overset{||}{C}}—OR_4 \qquad (III)$$

wherein X represents a halogen atom, $R_3$ represents an alkylene group having one to four carbon atoms, and $R_4$ represents a hydrogen atom or lower alkyl group, and then reacting the resulting reaction product

with a 4-hydroxy-2,2,6,6-tetramethylpiperidine compound represented by the formula (IV),

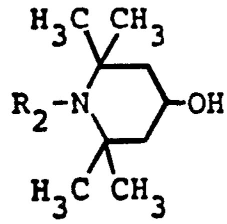

(IV)

wherein $R_2$ represents a hydrogen atom or methyl group, in the presence of a basic catalyst.

In the first-step reaction of this method, the molar ratio of 4-amino-2,2,6,6-tetramethylpiperidine compound represented by the formula (II) to halogenated carboxylic acid represented by the formula (III) is usually 1:1.5 to 1:3, preferably 1:2 to 1:2.5. This reaction proceeds without a catalyst, but it is preferred to use inert organic solvent such as toluene, xylene, etc. Also, dehydro-halogenating agents as represented by triethylamine, pyridine, etc. may be used. The reaction temperature is e.g. 10° to 150°C, preferably 10° to 100°C.

The halogenated carboxylic compounds useable in this reaction include for example monochloroacetic acid, monobromoacetic acid, 3-chloropropionic acid, 3-bromopropionic acid, 4-chlorobutyric acid, 4-bromobutyric acid, 5-chlorovaleric acid, 5-bromovaleric acid and the lower alkyl esters thereof (e.g. methyl, ethyl, propyl and butyl esters); the lower alkyl esters of the halogenated carboxylic acids are preferably used.

The second-step reaction is carried out by reacting the reaction product from the first step with a 4-hydroxy-2,2,6,6-tetramethylpiperidine compound represented by the formula (IV) in the presence of a basic catalyst. In this reaction, the reaction solution after completion of the first-step reaction may be used as it is, or the reaction product separated from it may be used.

In this reaction, the molar amount of 4-hydroxy-2,2,6,6-tetramethylpiperidine compound used is usually 1.5 to 4 times, preferably 2 to 2.5 times, that of the 4-amino-2,2,6,6-tetramethylpiperidine starting material.

This reaction proceeds without a solvent, but it is preferred to use inert organic solvent such as methanol, toluene, xylene, etc. Suitable basic catalysts are for example potassium hydroxide, sodium hydroxide, lithium hydroxide, lithium aluminum hydride, sodium boron hydride, sodium hydride, lithium hydride, sodium amide, sodium tert-butoxide, potassium tert-butoxide, sodium methoxide, potassium methoxide, sodium phenoxide, potassium phenoxide, metallic sodium, and metallic potassium. Of these, potassium tert-butoxide, sodium methoxide, sodium phenoxide and sodium hydroxide are preferably used. The molar amount of catalyst is e.g. 0.01 to 1 times, preferably 0.1 to 0.5 times, that of the 4-amino-2,2,6,6-tetramethylpiperidine starting material.

The reaction temperature is e.g. 0° to 150°C, preferably 10° to 50°C.

Typical 2,2,6,6-tetramethylpiperidine derivatives thus produced are shown in Table 1. In the method of the present invention, the monoester derivative is sometimes formed together with the diester derivative, but this mixture may be used as such for resin stabilization etc.

Table 1

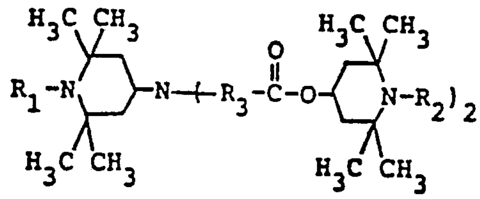

| Compound No. | $R_1$ | $R_2$ | $R_3$ |
|---|---|---|---|
| 1 - 1 | H | H | $CH_2$ |
| 1 - 2 | H | $CH_3$ | $CH_2$ |
| 1 - 3 | $CH_3$ | $CH_3$ | $CH_2$ |
| 1 - 4 | H | H | $CH_2CH_2$ |
| 1 - 5 | H | H | $CH_2CH_2CH_2$ |
| 1 - 6 | H | H | $CH_2CH_2CH_2CH_2$ |

When 2,2,6,6-tetramethylpiperidine derivative according to the present invention is used for resin stabilization, the amount blended with the synthetic resin is usually 0.01 to 5 parts by weight, preferably 0.05 to 2 parts by weight based on 100 parts by weight of the synthetic resin. For the blending, well-known apparatus and methods for incorporating stabilizers, pigments, fillers, etc. in synthetic resins may be used.

The resin stabilizers of the present invention may be used with other additives such as antioxidants, photostabilizers, metal sequestering agents, metal soaps, nucleating agents, lubricants, antistatic agents, flame retardants, pigments, and fillers.

Thus thermal stability and oxidation stability of synthetic resins can be improved by using a phenol antioxidant.

Suitable phenol antioxidants include for example 2,6-di-tert-butyl-4-methylphenol, n-octadecyl β-(3,5-di-tert-butyl-4-hydroxyphenyl)propionate, 1,1,3-tris(2-methyl-4-hydroxy-5-tert-butylphenyl)butane, 1,3,5-trimethyl-2,4,6-tris(3,5-di-tert-butyl-4-hydroxybenzyl)benzene, 1,3,5-tris(3,5-di-tert-butyl-4-hydroxybenzyl)-isocyanurate, 1,3,5-tris[β-(3,5-di-tert-butyl-4-hydroxyphenyl)propionyloxyethyl]isocyanurate, 1,3,5-tris(2,6-di-methyl-3-hydroxy-4-tert-butylbenzyl)isocyanurate and pentaerythritol tetrakis[β-(3,5-di-tert-butyl-4-hydroxyphenyl)propionate].

Also, color can be improved by using a phosphite antioxidant; suitable such antioxidants include tris-(nonylphenyl)phosphite, distearyl pentaerythritol diphosphite, tris(2,4-di-tert-butylphenyl)phosphite, tris(2-tert-butyl-4-methylphenyl)phosphite, bis(2,4-di-tert-butylphenyl)pentaerythritol diphosphite, and tetrakis-(2,4-di-tert-butylphenyl)-4,4'-biphenylene diphosphite.

When conventional hindered amine type light stabilizers are used in combination with sulfur-containing antioxidants, their light-resistance performance is remarkably decreased. In contrast, compounds of this invention show less such decrease in light-resistant performance and can be used in combination with known sulfur-containing antioxidants. Examples of such sulfur-containing antioxidants are dilauryl thiodipropionate, dimyristyl thiodipropionate, distearyl thiodipropionate, pentaerythritol tetrakis(β-laurylthiopropionate), and pentaerythritol tetrakis(β-hexylthiopropionate).

Synthetic resins which may be stabilized according to the present invention include for example low-density polyethylene, high-density polyethylene, linear low-density polyethylene, chlorinated polyethylene, EVA resin, polypropylene, polyvinyl chloride, methacrylic resin, polystyrene, impact-resistant polystyrene, ABS resin, AES resin, MBS resin, polyethylene terephthalate, polybutylene terephthalate, polyamide, polyimide, polycarbonate, polyacetal, polyurethane, and unsaturated polyester resin.

The invention is illustrated in detail by the following Examples and Comparative Examples, which are not however to be interpreted as limiting the invention.

Example 1

Production of Compound 1—1

A solution of 10.0 g (0.064 mole) of 4-amino-2,2,6,6-tetramethylpiperidine in 60 ml of toluene was kept at 60°C, 16.1 g (0.13 mole) of ethyl monochloroacetate was added over 10 minutes, and the solution was then kept at 80°C for 1 hour. After completion of the reaction, the reaction solution was washed with an aqueous saturated sodium hydrogencarbonate solution and then with water, and the toluene layer was concentrated to obtain 19.7 g of a transparent oily liquor.

4.6 Grams (0.014 mole) of this oily liquor and 5.1 g (0.032 mole) of 4-hydroxy-2,2,6,6-tetramethylpiperidine were dissolved in 50 ml of methanol, and 1.2 g (0.0064 mole) of 28% sodium methylate was added to the resulting solution which was then refluxed for 2 hours.

After completion of the reaction, the solvent was removed by evaporation, and the residue was diluted with 30 ml of toluene and poured into 30 ml of ice water. After phase separation and washing with water, the solvent was removed by evaporation to obtain a pale brown crude crystal. This crude crystal was recrystallized from an ethyl acetate/hexane mixed solvent to obtain 6.9 g (purity 80%) of a white crystal. By three recrystallizations from n-hexane there was obtained the desired product in the form of white crystals (purity 97.5%, m.p. 108—109°C).

Molecular ion peak of mass spectrum, 550

H'—NMR spectrum:

δ = 5.2 (t, 2H), 3.6 (s, 4H), 3.2 (t, 1H), 1.9 (d, 4H), 1.8 (d, 2H), 1.2 (m, 45H)

Elementary analysis (as $C_{31}H_{58}N_4O_4$):

| | C | H | N |
|---|---|---|---|
| Found (%) | 67.70 | 10.70 | 10.05 |
| Calculated (%) | 67.58 | 10.63 | 10.17 |

Example 2

Production of Compound 1—3

10.0 Grams (0.059 mole) of 1-methyl-4-amino-2,2,6,6-tetramethylpiperidine was dissolved in 60 ml of toluene and reacted with 16.1 g (0.13 mole) of ethyl monochloroacetate in the same manner as in Example 1. The reaction solution obtained was similarly after-treated to obtain 17.7 g of a transparent oily liquor.

4.8 Grams (0.014 mole) of this oily liquor was reacted with 5.5 g (0.032 mole) of 1-methyl-4-hydroxy-2,2,6,6-tetramethylpiperidine and then purified in the same manner as in Example 1 to obtain 7.8 g of white crystals as final product.

Yield, 82%; m.p., 85°—88°C.

Molecular ion peak of mass spectrum, 592

Elementary analysis (as $C_{34}H_{64}N_4O_4$):

| | C | H | N |
|---|---|---|---|
| Found (%) | 62.56 | 9.80 | 9.61 |
| Calculated (%) | 62.78 | 9.88 | 9.45 |

Example 3

Production of Compound 1—5

10.0 Grams (0.064 mole) of 4-amino-2,2,6,6-tetramethylpiperidine was dissolved in 60 ml of toluene and reacted with 19.6 g (0.13 mole) of ethyl 4-chlorobutyrate in the same manner as in Example 1. The reaction solution obtained was similarly after-treated to obtain 21.6 g of a transparent oily liquor.

5.0 Grams (0.014 mole) of this oily liquor was reacted with 5.1 g (0.032 mole) of 4-hydroxy-2,2,6,6-tetramethylpiperidine and then purified in the same manner as in Example 1 to obtain 7.0 g of white crystals as final product.

Yield, 80%; m.p., 90°—93°C.

Molecular ion peak of mass spectrum, 592

Elementary analysis (as $C_{34}H_{64}N_4O_4$):

| | C | H | N |
|---|---|---|---|
| Found (%) | 62.89 | 9.70 | 9.40 |
| Calculated (%) | 62.78 | 9.88 | 9.45 |

Example 4

The components listed below were mixed on a mixer for 5 minutes and then melt-kneaded at 180°C on a mixing roll to obtain a blend. This blend was then formed into sheet 1 mm thick on a hot press kept at 210°C; a test piece 150 × 130 × 1 mm in size was prepared from the sheet. This was repeated using different test compounds.

Each test piece was exposed to light in a Sunshine weather-O-meter (light source, carbon arc; temperature of black panel, 83°±3°C; spraying cycle, 120 minutes; spraying time, 18 minutes) and folded every 60 hours to obtain the time required for the test piece to break in two. The weathering resistance was

evaluated by this time.

Further, a 40 × 40 × 1 mm test piece was prepared, and in a Geer oven at 160°C, the time until 30% of the test piece area became brittle was measured; this time was taken as the "Induction period to embrittlement", by which the heat and oxidation stability was evaluated.

The results are shwon in Table 2.

| *Blend* | Parts by weight |
|---|---|
| Unstabilized polypropylene | 100 |
| Calcium stearate | 0.1 |
| 2,6-Di-tert-butyl-4-methylphenol | 0.05 |
| Test compound | As in Table 2 |

In Tables 2 to 4, UVA—1 to UVA—8, AO—1 and AO—2 mean the following compounds:

| | |
|---|---|
| UVA—1 | : 2-Hydroxy-4-methoxybenzophenone |
| UVA—2 | : 2-Hydroxy-4-n-octoxybenzophenone |
| UVA—3 | : 2-(2-Hydroxy-5-methylphenyl)benzotriazole |
| UVA—4 | : 2-(2-Hydroxy-3-tert-butyl-5-methylphenyl)-5-chlorobenzotriazole |
| UVA—5 | : 2-(2-Hydroxy-3,5-dipentylphenyl)benzotriazole |
| UVA—6 | : ethyl 2-cyano-3,3'-diphenylacrylate |
| UVA—7 | : Nickel salt of bis(3,5-di-tert-butyl-4-hydroxybenzylphosphoric acid) monoethyl ester |
| UVA—8 | : Bis(2,2,6,6-tetramethyl-4-piperidyl)sebacate |
| AO—1 | : Tris(3,5-di-tert-butyl-4-hydroxybenzyl)isocyanurate |
| AO—2 | : Dilauryl-3,3'-thiodipropionate |

Table 2

| Test compound | Example 1 | 2 | 3 | 4 | 5 | 6 | 7 | 8 | 9 | 10 | 11 | 12 |
|---|---|---|---|---|---|---|---|---|---|---|---|---|
| 1 - 1 | 0.2 | | | | | | 0.2 | | | | | |
| 1 - 2 | | 0.2 | | | | | | 0.2 | | | | |
| 1 - 3 | | | 0.2 | | | | | | 0.2 | | | |
| 1 - 4 | | | | 0.2 | | | | | | 0.2 | | |
| 1 - 5 | | | | | 0.2 | | | | | | 0.2 | |
| 1 - 6 | | | | | | 0.2 | | | | | | 0.2 |
| AO - 1 | | | | | | | 0.05 | 0.05 | 0.05 | 0.05 | 0.05 | 0.05 |
| AO - 2 | | | | | | | 0.25 | 0.25 | 0.25 | 0.25 | 0.25 | 0.25 |
| Weathering resistance (hours) | 2220 | 2160 | 2040 | 2160 | 2100 | 1980 | 1920 | 1860 | 1740 | 1860 | 1800 | 1680 |
| Induction period to embrittlement (hours) | 60 | 60 | 55 | 60 | 55 | 55 | 780 | 715 | 680 | 710 | 690 | 675 |

Table 2 (cont'd)

| Test compound | Comparative example 13 | 14 | 15 | 16 | 17 | 18 | 19 | 20 | 21 | 22 | 23 | 24 | 25 | 26 | 27 | 28 | 29 |
|---|---|---|---|---|---|---|---|---|---|---|---|---|---|---|---|---|---|
| UVA - 1 | 0.2 | | | | | | | | 0.2 | | | | | | | | No addition |
| UVA - 2 | | 0.2 | | | | | | | | 0.2 | | | | | | | |
| UVA - 3 | | | 0.2 | | | | | | | | 0.2 | | | | | | |
| UVA - 4 | | | | 0.2 | | | | | | | | 0.2 | | | | | |
| UVA - 5 | | | | | 0.2 | | | | | | | | 0.2 | | | | |
| UVA - 6 | | | | | | 0.2 | | | | | | | | 0.2 | | | |
| UVA - 7 | | | | | | | 0.2 | | | | | | | | 0.2 | | |
| UVA - 8 | | | | | | | | 0.2 | | | | | | | | 0.2 | |
| AO - 1 | | | | | | | | | 0.05 | 0.05 | 0.05 | 0.05 | 0.05 | 0.05 | 0.05 | 0.05 | |
| AO - 2 | | | | | | | | | 0.25 | 0.25 | 0.25 | 0.25 | 0.25 | 0.25 | 0.25 | 0.25 | |
| Weathering resistance (hours) | 180 | 420 | 240 | 600 | 420 | 240 | 360 | 1800 | 240 | 480 | 360 | 720 | 480 | 240 | 420 | 960 | 120 |
| Induction period to embrittlement (hours) | 20 | 30 | 20 | 45 | 30 | 20 | 30 | 30 | 480 | 490 | 480 | 500 | 490 | 480 | 485 | 485 | 5 |

Example 5

To respective lots of 25% urethane dope (comprising 25 parts of a polyurethane resin, 3.75 parts of dimethylformamide and 71.25 parts of tetrahydrofuran) were added the test compounds shown in Table 3 at a rate of 1% based on the polyurethane resin. Each mixture was coated onto polyester film in a thickness of 1.2 mm and dried for 1 hour in a dryer kept at 45°C. The sheets thus obtained were punched into No. 3 dumb-bell test pieces. The test pieces were exposed to light for 60 hours and 120 hours in a fade-O-meter (light source: ultraviolet carbon arc, temperature of black panel: 63°±3°C), and a percent retention of break strength was obtained by the tensile test (tensile rate: 200 mm/mm, measurement temperature: 25°C).

The results are shown in Table 3.

Table 3

| Test Compound | Example 1 | 2 | 3 | 4 | 5 | 6 | 7 | 8 | 9 | 10 | 11 | 12 | Comparative example 13 | 14 | 15 | 16 | 17 | 18 | 19 |
|---|---|---|---|---|---|---|---|---|---|---|---|---|---|---|---|---|---|---|---|
| 1 - 1 | 1 | | | | | | 0.7 | | | | | | | | | | | | |
| 1 - 2 | | 1 | | | | | | 0.7 | | | | | | | | | | | |
| 1 - 3 | | | 1 | | | | | | 0.7 | | | | | | | | | | |
| 1 - 4 | | | | 1 | | | | | | 0.7 | | | | | | | | | |
| 1 - 5 | | | | | 1 | | | | | | 0.7 | | | | | | | | |
| 1 - 6 | | | | | | 1 | | | | | | 0.7 | | | | | | | |
| UVA - 2 | | | | | | | | | | | | | 1 | | | 0.7 | | | |
| UVA - 5 | | | | | | | | | | | | | | 1 | | | 0.7 | | No additon |
| UVA - 8 | | | | | | | | | | | | | | | 1 | | | 0.7 | |
| AO - 1 | | | | | | | 0.05 | 0.05 | 0.05 | 0.05 | 0.05 | 0.05 | | | | 0.05 | 0.05 | 0.05 | |
| AO - 2 | | | | | | | 0.25 | 0.25 | 0.25 | 0.25 | 0.25 | 0.25 | | | | 0.25 | 0.25 | 0.25 | |
| Percent retention of break strength 60 hrs. | 85 | 82 | 78 | 81 | 79 | 72 | 81 | 78 | 74 | 77 | 75 | 70 | 43 | 56 | 66 | 41 | 53 | 56 | 30 |
| Percent retention of break strength 120 hrs. | 65 | 60 | 56 | 59 | 57 | 47 | 62 | 57 | 53 | 56 | 54 | 45 | 22 | 30 | 40 | 21 | 28 | 33 | 16 |

Example 6

The blend described below was melt-kneaded on a mixing roll kept at 150°C and then formed into a sheet 0.5 mm thick on a hot press kept at 160°C. This was repeated using different test compounds.

The sheets were exposed to light for 1200 hours in a Sunshine weather-o-meter (light source: carbon arc, temperature of black panel: 63°±3°C, spraying cycle: 120 minutes, spraying time: 18 minutes), and the degree of discoloration was observed.

The results are shown in Table 4.

| *Blend* | Parts by weight |
|---|---|
| Polyvinyl chloride | 100 |
| Dioctyl phthalate | 38 |
| Epoxidized soybean oil | 2 |
| Barium stearate | 1 |
| Zinc stearate | 0.3 |
| Test compound | 0.2 |

Table 4

| | No. | Test compound | Degree of discoloration |
|---|---|---|---|
| Example | 1 | 1 - 1 | Pale yellow |
| | 2 | 1 - 2 | " |
| | 3 | 1 - 3 | " |
| | 4 | 1 - 4 | " |
| | 5 | 1 - 5 | " |
| | 6 | 1 - 6 | " |
| Comparative Example | 7 | UVA - 2 | Brown spot |
| | 8 | UVA - 3 | Yellow |
| | 9 | UVA - 8 | Yellow |
| | 10 | No addition | Blackish brown |

**Claims**

1. A 2,2,6,6-tetramethylpiperidine derivative represented by the formula (I),

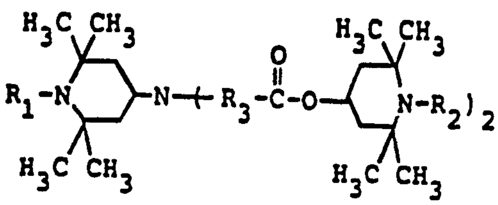

(I)

wherein $R_1$ and $R_2$ independently represent a hydrogen atom or methyl group, and $R_3$ represents an alkylene group having one to four carbon atoms.

2. A derivative according to Claim 1 wherein each of $R_1$ and $R_2$ is a hydrogen atom.

3. A derivative according to Claim 1 or 2 whereinc $R_3$ is $—CH_2$, $—CH_2CH_2—$, $—CH_2CH_2CH_2—$ or $—CH_2CH_2CH_2CH_2—$.

4. A method for producing a 2,2,6,6-tetramethylpiperidine derivative according to claim 1 which comprises reacting, preferably at 10—100°C, a compound of formula (II),

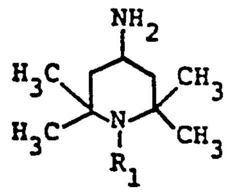

(II)

with a halogenated carboxylic compound of formula (III),

$$X—R_3—\overset{\overset{O}{\|}}{C}—OR_4 \qquad (III)$$

and then reacting the resulting reaction product, e.g. at 10 to 50°C and after or without separation from the reacted mixture, with a 4-hydroxy-2,2,6,6-tetramethylpiperidine compound of formula (IV),

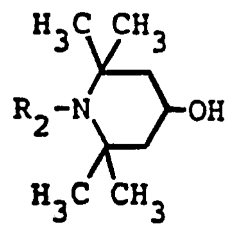

(IV)

in the presence of a basic catalyst, wherein $R_1$, $R_2$ and $R_3$ are as defined in claim 1, X is a halogen atom, and $R_4$ is a hydrogen atom or lower alkyl group.

5. A method according to claim 4 wherein the molar ratio of compound (II) to compound (III) used is 1:1.5 to 1:3, preferably 1:2 to 1:2.5.

6. A method according to claim 4 or 5 wherein the formula (III) compound is selected from monochloroacetic acid, monobromoacetic acid, 3-chloropropionic acid, 3-bromopropionic acid, 4-chlorobutyric acid, 4-bromobutyric acid, 5-chlorovaleric acid, 5-bromovaleric acid and lower alkyl esters thereof such as methyl, ethyl, propyl and butyl esters.

7. A method according to any of claims 4 to 6 wherein formula (IV) compound is used in a molar amount of 1.5 to 4 times, preferably 2 to 2.5 times, that of the formula (II) starting compound.

8. A method according to any of claims 4 to 7 wherein the basic catalyst, selected for example from sodium hydroxide, potassium hydroxide, lithium hydroxide, lithium aluminum hydride, sodium boron hydride, sodium hydride, lithium hydride, sodium amide, sodium tert-butoxide, potassium tert-butoxide, sodium methoxide, potassium methoxide, sodium phenoxide, potassium phenoxide, metallic sodium and metallic potassium is used in a molar amount of 0.1 to 1 times, preferably 0.1 to 0.5 times, that of the formula (II) starting compound.

9. A method according to any of claims 4 to 8 wherein one or both of the two reaction steps is conducted in inert organic solvent.

10. A derivative according to any of claims 1 to 3 in admixture with monoester derivative of formula

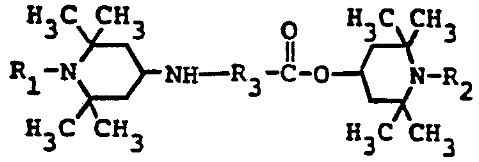

wherein $R_1$, $R_2$ and $R_3$ are as defined in claim 1.

11. A derivative according to any of claims 1 to 3 and 10 in admixture with at least one synthetic resin additive selected from other photostabilizers, antioxidants (including phenolic, phosphite and sulfur-containing anti-oxidants), metal sequestering agents, metal soaps, nucleating agents, lubricants, antistatic

agents, flame retardants, pigments and fillers.

12. Synthetic resin, selected from low-density polyethylene, high-density polyethylene, linea low-density polyethylene, chlorinated polyethylene, EVA resin, polypropylene, polyvinyl chloride, methacrylic resin, polystyrene, impact-resistant polystyrene, ABS resin, AES resin, MBS resin, polyethylene terephthalate, polybutylene terephthalate, polyamide, polyimide, polycarbonate, polyacetal, polyurethane and unsaturated polyester resin, incorporating as a stabiliser a derivative according to any of claims 1 to 3, 10 and 11.

## Patentansprüche

1. 2,2,6,6-Tetramethylpiperidin-Derivat der Formel (I),

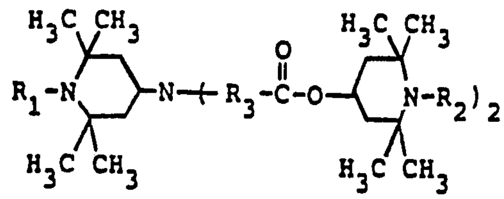

(I)

worin $R_1$ und $R_2$ unabhängig voneinander ein Wasserstoffatom oder eine Methylgruppe und $R_3$ eine Alkylengruppe mit 1 bis 4 Kohlenstoffatomen bedeuten.

2. Derivat nach Anspruch 1, dadurch gekennzeichnet, daß jeder der Reste $R_1$ und $R_2$ ein Wasserstoffatom ist.

3. Derivat nach Anspruch 1 oder 2, dadurch gekennzeichnet, daß $R_3$ die Gruppe $—CH_2—$, $—CH_2CH_2—$, $—CH_2CH_2CH_2—$ oder $—CH_2CH_2CH_2CH_2—$ ist.

4. Verfahren zur Herstellung eines 2,2,6,6-Tetramethylpiperidin-Derivats nach Anspruch 1, dadurch gekennzeichnet, daß man, vorzugsweise bei 10 bis 100°C, eine Verbindung der Formel (II)

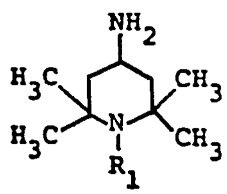

(II)

mit einer halogenierten Carbonsäureverbindung der Formel (III)

$$X—R_3—\overset{\overset{\displaystyle O}{\|}}{C}—OR_4 \qquad (III)$$

umsetzt und dann das erhaltene Reaktionsprodukt, z.B. bei 10 bis 50°C und ohne oder nach Abtrennung vom Reaktionsgemisch mit einer 4-Hydroxy-2,2,6,6-Tetramethylpiperidinverbindung der Formel (IV)

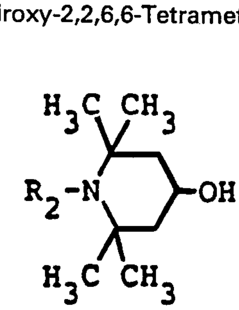

(IV)

in Gegenwart eines basischen Katalysators umsetzt, wobei $R_1$, $R_2$ und $R_3$ die in Anspruch 1 angegebenen Bedeutungen haben, X ein Halogenatom ist und $R_4$ ein Wasserstoffatom oder eine niedere Alkylgruppe ist.

5. Verfahren nach Anspruch 4, dadurch gekennzeichnet, daß das angewandte molare Verhältnis an Verbindung (II) zur Verbindung (III) 1:1,5 bis 1:3, vorzugsweise 1:2 bis 1:2,5 beträgt.

6. Verfahren nach Anspruch 4 oder 5, dadurch gekennzeichnet, daß die Verbindung der Formel (III) aus der Gruppe Monochloressigsäure, Monobromessigsäure, 3-Chlorpropionsäure, 3-Brompropionsäure, 4-Chlorbuttersäure, 4-Brombuttersäure, 5-Chlorvaleriansäure, 5-Bromvaleriansäure und niedrigen Alkylestern davon, wie Methyl-, Ethyl-, Propyl- und Butylestern, ausgewählt ist.

7. Verfahren nach einem der Ansprüche 4 bis 6, dadurch gekennzeichnet, daß die Verbindung der Formel (IV) in einer molaren Menge des 1,5- bis 4-fachen, vorzugsweise 2- bis 2,5-fachen derjenigen der Ausgangsverbindung der Formel (II) verwendet wird.

8. Verfahren nach einem der Ansprüche 4 bis 7, dadurch gekennzeichnet, daß der basische Katalysator, der beispielsweise aus Natriumhydroxid, Kaliumhydroxid, Lithiumhydroxid, Lithium-Aluminiumhydrid, Natrium-Borhydrid, Natriumhydrid, Lithiumhydrid, Natriumamid, Natrium-tert-butylat, Kalium-tert-butylat, Natriummethylat, Kaliummethylat, Natriumphenolat, Kaliumphenolat, metallischem Natrium und metallischem Kalium gewählt sein kann, in einer molaren Menge des 0,1- bis 1-fachen, vorzugsweise 0,1- bis 0,5-fachen derjenigen der Ausgangsverbindung der Formel (II) eingesetzt wird.

9. Verfahren nach einem der Ansprüche 4 bis 8, dadurch gekennzeichnet, daß eine oder beide der zwei Reaktionsstufen in einem inerten organischen Lösungsmittel durchgeführt werden.

10. Derivat nach einem der Ansprüche 1 bis 3 in Mischung mit Monoesterderivaten der Formel

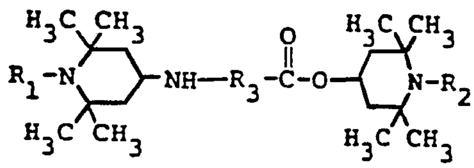

worin $R_1$, $R_2$ und $R_3$ die in Anspruch 1 angegebenen Bedeutungen haben.

11. Derivat nach einem der Ansprüche 1 bis 3 und 10 in Mischung mit wenigstens einem Zusatz für synthetische Harze aus der Gruppe andere Photostabilisatoren, Antioxidatien (einschließlich von phenolischen, Phosphit- und schwefelhaltigen Antioxidatien), Metallkomplexe bildenden Mitteln, Metallseifen, keimbildenden Mitteln, Schmierstoffen, Antistatikmitteln, Flammverzögerern, Pigmenten und Füllstoffen.

12. Synthetisches Harz, ausgewählt aus Polyethylen niederer Dichte, Polyethylen hoher Dichte, linearem Polyethylen niederer Dichte, chloriertem Polyethylen, EVA-Harz, Polypropylen, Polyvinylchlorid, Methacrylharz, Polystyrol, schlagzähem, Polystyrol, ABS-Harz, AES-Harz, MBS-Harz, Polyethylenterephthalat, Polybutylenterephthalat, Polyamid, Polyimid, Polycarbonat, Polyacetal, Polyurethan und ungesättigtem Polyesterharz, das als Stabilisator ein Derivat nach einem der Ansprüche 1 bis 3, 10 und 11 enthält.

**Revendications**

1. Dérivé de 2,2,6,6-tétraméthylpipéridine représenté par la formule (I)

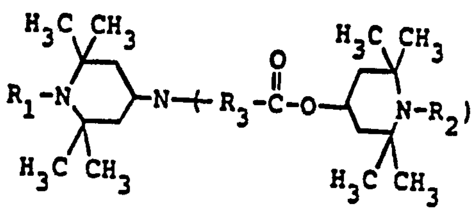

(I)

dans laquelle $R_1$ et $R_2$ représentent, indépendamment, un atome d'hydrogène ou un groupe méthyle, et $R_3$ représente un groupe alkylène ayant un à quatre atoms de carbone.

2. Dérivé suivant la revendication 1, dans lequel chacun de $R_1$ et $R_2$ est un atome d'hydrogène.

3. Dérivé suivant la revendication 1 ou 2, dans lequel $R_3$ représente $—CH_2—$, $—CH_2CH_2—$, $—CH_2CH_2CH_2—$ ou $—CH_2CH_2CH_2CH_2—$.

4. Procédé de production d'un dérivé de 2,2,6,6-tétraméthylpipéridine suivant la revendication 1, qui consiste à faire réagir, de préférence à 10—100°C, un composé de formule (II)

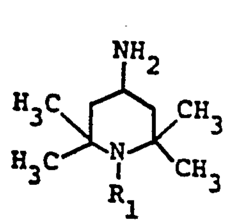

(II)

avec un composé carboxylique halogéne de formule (III)

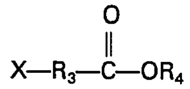 (III)

puis à faire réagir le produit réactionnel résultant, par exemple à 10—50°C et après ou sans séparation du mélange ayant réagi, avec un composé de 4-hydroxy-2,2,6,6-tétraméthylpipéridine de formule (IV)

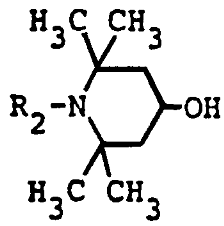 (IV)

en présence d'un catalyseur basique, $R_1$, $R_2$ et $R_3$ étant tels que définis dans la revendication 1, X représentant un atome d'halogène et $R_4$ étant un atome d'hydrogène ou un groupe alkyle inférieur.

5. Procédé suivant la revendication 4, dans lequel le rapport molaire du composé (II) au composé (III) utilisé a une valeur de 1:1,5 à 1:3, de préférence de 1:2 à 1:2,5.

6. Procédé suivant la revendication 4 ou 5, dans lequel le composé de formule (III) est choisi entre l'acide monochloracétique, l'acide monobromacétique, l'acide 3-chlorpropionique, l'acide 3-bromo-propionique, l'acide 4-chlorobutyrique, l'acide 4-bromobutyrique, l'acide 5-chlorovalérique, l'acide 5-bromovalérique et leurs esters alkyliques inférieurs tels que leurs esters de méthyle, d'éthyle, de propyle et de butyle.

7. Procédé suivant l'une quelconque des revendications 4 à 6, dans lequel le composé de formule (IV) est utilisé en une quantité molaire de 1,5 à 4 fois, de préférence de 2 à 2,5 fois la quantité de composé de départ de formule (II).

8. Procédé suivant l'une quelconque des revendications 4 à 7, dans lequel le catalyseur basique, choisi par exemple entre l'hydroxyde de sodium, l'hydroxyde de potassium, l'hydroxyde de lithium, l'hydrure de lithium et d'aluminium, l'hydrure de sodium et de bore, l'hydrure de sodium, l'hydrure de lithium, un amidure de sodium, le tertio-butylate de sodium, le tertio-butylate de potassium, le méthylate de sodium, le méthylate de potassium, le phénate de sodium, le phénate de potassium, le sodium métallique et le potassium métallique, est utilisé en une quantité molaire de 0,1 à 1 fois, de préférence de 0,1 à 0,5 fois la quantité de composé de départ de formule (II).

9. Procédé suivant l'une quelconque des revendications 4 à 8, dans lequel on conduit l'une ou l'autre de ces deux étapes réactionnelles ou les deux dans un solvant organique inerte.

10. Dérivé suivant l'une quelconque des revendications 1 à 3, en mélange avec un mono-ester de formule

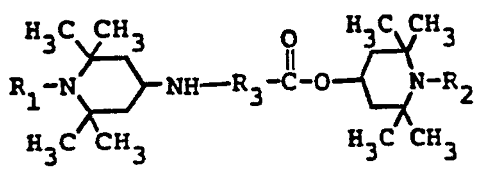

dans laquelle $R_1$, $R_2$ et $R_3$ sont tels que définis dans la revendication 1.

11. Dérivé suivant l'une quelconque des revendications 1 à 3 et 10, en mélange avec au moins un additif pour résine synthétique choisi entre d'autres agents photostabilisants, des anti-oxydants (comprenant des anti-oxydants phénoliques, phosphitiques et contenant du soufre), des agents de séquestration des métaux, des savons métalliques, des agents de nucléation, des lubrifiants, des agents antistatiques, des retardateurs de flamme, des pigments et des charges.

12. Résine synthétique, choisie entre un polyéthylène basse densité, un polyéthylène haute densité, un polyéthylène linéaire basse densité, un polyéthylène chloré, une résine EVA, un polypropylène, un polychlorure de vinyle, une résine méthacrylique, un polystyrène, un polystyrène résistant au choc, une résine ABS, une résine AES, une résine MBS, un polytéréphtalate d'éthylène, un polytéréphtalate de butylène, un polyamide, un polyimide, un polycarbonate, un polyacétal, un polyuréthanne et une résine polyester non saturée, contenant comme agent stabilisant un dérivé suivant l'une quelconque des revendications 1 à 3, 10 et 11.